(19) Europäisches Patentamt / European Patent Office / Office européen des brevets

(11) **EP 2 845 599 B1**

(12) **EUROPEAN PATENT SPECIFICATION**

(45) Date of publication and mention
of the grant of the patent:
**22.03.2017 Bulletin 2017/12**

(51) Int Cl.:
*A61K 9/51* (2006.01)    *A61K 35/36* (2015.01)
*A61K 35/55* (2015.01)    *A61K 35/413* (2015.01)
*A61K 33/36* (2006.01)    *A61K 36/324* (2006.01)
*A61K 36/328* (2006.01)    *A61P 35/00* (2006.01)

(21) Application number: **14780742.4**

(22) Date of filing: **13.02.2014**

(86) International application number:
**PCT/CN2014/072026**

(87) International publication number:
**WO 2014/206093 (31.12.2014 Gazette 2014/53)**

(54) **CHINESE HERBAL COMPOUND ANTI-TUMOUR NANOPREPARATION AND USE THEREOF**

ANTITUMORALES NANOPRÄPARAT AUS EINER CHINESISCHEN PFLANZLICHEN VERBINDUNG UND VERWENDUNG DAVON

NANOPRÉPARATION ANTITUMORALE DE COMPOSÉ À BASE DE PLANTES CHINOIS ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.06.2013 CN 201310258335**

(43) Date of publication of application:
**11.03.2015 Bulletin 2015/11**

(73) Proprietor: **Shanghai University of Traditional Chinese Medicine**
**Shanghai 201203 (CN)**

(72) Inventors:
• **FENG, Nianping**
  **Shanghai 201203 (CN)**
• **SHI, Feng**
  **Shanghai 201203 (CN)**
• **LIU, Ying**
  **Shanghai 201203 (CN)**
• **ZHAO, Jihui**
  **Shanghai 201203 (CN)**
• **ZHANG, Yongtai**
  **Shanghai 201203 (CN)**

(74) Representative: **Sun, Yiming**
**HUASUN Patent- und Rechtsanwälte**
**Friedrichstraße 33**
**80801 München (DE)**

(56) References cited:
**WO-A1-2006/128634**    **CN-A- 1 362 141**
**CN-A- 1 368 294**    **CN-A- 101 991 788**

• **NIANPING FENG: "Preparation and characterization of solid lipid nanoparticles loaded with frankincense and myrrh oil", INTERNATIONAL JOURNAL OF NANOMEDICINE, 1 April 2012 (2012-04-01), page 2033, XP055183417, ISSN: 1176-9114, DOI: 10.2147/IJN.S30085**
• **DATABASE WPI Week 201147 Thomson Scientific, London, GB; AN 2011-H11583 XP002738635, & CN 102 058 747 A (YANGLING DOMHKE MAIDISEN PHARM CO LTD) 18 May 2011 (2011-05-18)**
• **DATABASE BIOSIS [Online] BIOSCIENCES INFORMATION SERVICE, PHILADELPHIA, PA, US; January 2012 (2012-01), CHENG MIAO ET AL: "Study on the Effect of Realgar Nanoparticles on Reducing the Respiratory Syncytial Virus Type A (RSV-A) Replication in Vitro", XP002738636, Database accession no. PREV201200243880 & CHINESE JOURNAL OF VIROLOGY, vol. 28, no. 1, January 2012 (2012-01), pages 45-50, ISSN: 1000-8721**

**(Cont. next page)**

- **LIU ET AL: "Development of Chinese medicine based on pharmacology and therapeutics", JOURNAL OF ETHNOPHARMACOLOGY, ELSEVIER IRELAND LTD, IE, vol. 19, no. 2, 1 March 1987 (1987-03-01), pages 119-123, XP025579175, ISSN: 0378-8741, DOI: 10.1016/0378-8741(87)90035-3 [retrieved on 1987-03-01]**
- **JIN, FEN ET AL.: 'Optimization of the Extraction Process of Mastic and Myrrh in NIUHUANGXINGXIAOWAN' HUBEI JOURNAL OF TCM vol. 31, no. 05, 10 May 2009, pages 58 - 59, XP008175064**
- **FENG, NIANPING ET AL.: 'Discussion on Nano-delivery Systems for Traditonal Chinese Drugs' WORLD SCIENCE AND TECHNOLOGY / MODERNIZATION OF TRADITIONAL CHINESE MEDICINE AND MATERIA MEDICA vol. 5, no. 04, 30 October 2003, pages 52 - 55, XP008175128**

**Description**

**FIELD OF THE INVENTION**

The present invention relates to the field of medical technology, specifically to a traditional Chinese medicine antitumor composition and use thereof.

**BACKGROUND OF THE INVENTION**

At present, the nanotechnology research of traditional Chinese medicine has made certain achievements. Through application of technologies including the nano-crystal technology, liposome, micro-emulsion, self-microemulsion, solid lipid nano-particles and so on, gratifying effect in study of a number of traditional Chinese medicine monomer component and effective parts is achieved. However, there are few nanotechnologies involved in the traditional Chinese medicine compound at present, the preparation of compound nano preparation in current nanotechnology literature is only achieved through simply crushing the whole compound medicinal granules to nanometer scale. Because of difference in physicochemical properties of medicinal materials of the traditional Chinese medicine compound, it is difficult to promote the above idea.

Nano-drugs are becoming an important way of treatment of malignant tumor. There are a substantial proportion of antitumor drugs out of nano drugs on the market. Nanotechnology improves the solubility of antitumor drugs that are difficult to dissolve and the selectivity to tumor cells, and reduces the toxic and side effects. Nano drugs have become a hotspot in the field of tumor treatment.

The compound Niu Huang Xing Xiao pill was first recorded in the *Life-saving Manual of Diagnosis and Treatment of External Diseases* written by a Chinese Qing Dynasty Doctor, WANG Hongxu, currently recorded in the departmental standard. The main medicines include bezoar, musk, frankincense (processed with vinegar), myrrh (processed with vinegar), realgar and so on. It is used for treatment of ulcer on the back, scrofula and flowing phlegm, acute mastitis and breast cancer, innominate inflammatory of unknown origin and so on, which are the equal of variety of malignant tumors in modern times. The prescription of clinical patent medicine on the market is as follows: 6g of bezoar, 30g of musk, 200g of frankincense (processed with vinegar), 200g of myrrh (processed with vinegar) and 100g of realgar. With hundreds of years of clinical experience, the compound Niu Huang Xing Xiao pill has undisputed curative effect, but the clinical dosage is large while the medicinal materials are expensive and precious; therefore, it is necessary to improve the dosage forms.

CN 1 362 141 discloses a nano Niuhuang-xingxiao preparation medicine, which uses 6 parts of nano bovine bezoar, 30 parts of nano nusk, 200 parts of nano frankiecense, 200 parts of nano myrrh and 100 part of nano realgar by weight as raw materials; the nano Niuhuang-xingxiao preparation medicine is made up through the steps of microwave extraction, concentration under reduced pressure and drying via supersonic jet spray, etc, and the grain fineness thereof is up to 1200-1500 meshes, and grain size is 0.1-200 nm, wherein most of grain size is less than 100 nm.

**SUMMARY OF THE INVENTION**

The technical problem to be solved in the present invention is to provide a traditional Chinese medicine antitumor composition. According to physicochemical property of the medicinal materials of the compound Niu Huang Xing Xiao pill, the preparation is divided into four components, that is, frankincense and myrrh volatile oil component (FMO), realgar component, artificial bezoar component and artificial musk component; according to physical and chemical property of each component, multiple-unit drug delivery systems are prepared with different nanotechnologies, and then the multiple-unit drug delivery systems are combined with reasonable method, thus to prepare the traditional Chinese medicine antitumor composition of the present invention. The preparation overcomes the shortcomings in the current process of preparing traditional Chinese medicine compound nano preparation, so the preparation method is more scientific and effective, and achieves better curative effect with reduced dosage.

In addition, the present invention further provides an use of the above traditional Chinese medicine antitumor composition.

In order to resolve the above technical problem, the present invention is achieved by the technical solution below:

In one aspect, the present invention provides a traditional Chinese medicine antitumor composition comprising the following components by weight percentage: 28%-32% of solid lipid nanoparticles containing volatile oil of frankincense and myrrh, 61%-65% of nano realgar carrying microcapsule, 0.8%-1.3% of bezoar micro-powder and 4%-6% of musk micro-powder.

The particle size of the solid lipid nanoparticles containing volatile oil of frankincense and myrrh is 40-300nm.

The particle size of the nano realgar is 80-200nm.

The particle size of the bezoar micro-powder is 1-20$\mu$m.

The particle size of the musk micro-powder is 5-50μm.

The solid lipid nanoparticles containing volatile oil of frankincense and myrrh is prepared with the following method: extracting the volatile oil in the frankincense and myrrh with steam distillation method at first, and then preparing the solid lipid nanoparticles containing volatile oil of frankincense and myrrh with high pressure homogenization method.

Preferably, extracting the volatile oil in the frankincense and myrrh with steam distillation method comprises the following steps: taking same amount of frankincense and myrrh respectively, crushing and mixing them together, extracting the volatile oil with water, and dehydrating the extracted volatile oil with anhydrous $Na_2SO_4$.

Preferably, preparing the solid lipid nanoparticles containing volatile oil of frankincense and myrrh with high pressure homogenization method comprises the following steps: heating the volatile oil of the frankincense and myrrh together with glyceryl behenate in a water bath, and obtaining oil phase after stirring and melting; placing and mixing same amount of soybean phospholipid and Tween-80 in a beaker, adding proper amount of distilled water into the beaker, stirring for the mixture to dissolve, and heating till the temperature is the same as that of the oil phase to obtain the water phase; adding the water phase into the oil phase, stirring to obtain the primary dispersed system, and then carrying out high pressure homogenization to obtain the solid lipid nanoparticles containing volatile oil of frankincense and myrrh.

The nano realgar carrying microcapsule is prepared with the following method: preparing nano realgar by using a high-energy ball mill with wet grinding method at first, mixing the nano realgar with gelatin solution uniformly, and then adding the gelatin solution containing the nano realgar into liquid paraffin, mixing in a water bath, cooling and continuously mixing in an ice-water bath, adding formaldehyde for curing, and then carrying out dehydration with isopropanol, after standing for a certain time, carrying out suction filtration, and washing with isopropanol and normal hexane, pumping and drying, thus to obtain the nano realgar carrying microcapsule.

Preferably, preparing nano realgar by using a high-energy ball mill with wet grinding method comprises the following steps: crushing block-shaped realgar and sieving to obtain coarse particles, adding the coarse particles into a ball mill, and adding saturated SDS solution there into as medium to carry out ball milling, taking the ball milled realgar out, carrying out water grind processing, thus to obtain the nano realgar.

The bezoar micro-powder is prepared by using a ball mill with dry grinding method.

The musk micro-powder is prepared with combination of liquid nitrogen refrigeration and super centrifugal grinding method.

The encapsulation efficiency of the mixed volatile oil of frankincense and myrrh in the solid lipid nanoparticles containing volatile oil of frankincense and myrrh is 50% or higher, the average encapsulation efficiency being $(80.60\pm1.11)\%$; the drug loading capacity of the mixed volatile oil of frankincense and myrrh is 40%-60%, the average drug loading capacity being $(53.73\pm0.74)\%$.

The encapsulation efficiency of the nano realgar in the nano realgar carrying microcapsule is 60% or higher, the average encapsulation efficiency being $(66.20\pm1.85)\%$; the drug loading capacity of the nano realgar is 20%-40%, the average drug loading capacity being $(26.30\pm1.14)\%$.

The dosage form of the traditional Chinese medicine antitumor composition includes capsule, granule or tablet.

In another aspect, the present invention further provides an use of the traditional Chinese medicine antitumor composition for preparing antitumor drugs.

The traditional Chinese medicine antitumor composition of the present invention improves dosage form of each medicine material of the compound Niu Huang Xing Xiao pill by using different nanotechnologies. The bezoar and musk are precious medicine materials, the bioavailability can be improved by using nanotechnology (grinding method), thereby reducing consumption of medicine materials. The main antitumor component of frankincense and myrrh is volatile oil, the irritation and stability can be improved by nano drug-loading technology (high pressure homogenization method). The realgar has both activity and toxicity, nanotechnology (wet grinding method by the high-energy ball mill) can be used to find a balance point to achieve better curative effect and lower toxicity. Animal experiments prove that the traditional Chinese medicine antitumor composition of the present invention, compared with the compound Niu Huang Xing Xiao pill on the market, has remarkable improvement on both bioavailability and tumor inhibitory effect in vivo.

**BRIEF DESCRIPTION OF THE DRAWINGS**

The following is detailed description of the present invention in combination with drawings and embodiments.

Fig.1 is the transmission electron microscope image of FMO-SLN of the present invention;

Fig.2 is the particle size and zeta potential diagram of FMO-SLN of the present invention;

Fig.3 is the transmission electron microscope image of nano realgar of the present invention;

Fig.4 is the microscopic examination image of the nano realgar carrying microcapsule of the present invention;

Fig.5 is As$_2$S$_2$ blood concentration - time curve after administration of the compound multiple- unit capsule of the embodiment 4 of the present invention and the Niu Huang Xing Xiao pill on the market.

## DETAILED DESCRIPTION OF THE INVENTION

The traditional Chinese medicine antitumor composition of the present invention, using the compound Niu Huang Xing Xiao pill as a model drug, according to physical and chemical properties of medicine materials of the formulation, based on different nanotechnologies, prepares multiple-unit drug delivery systems and combines the multiple-unit drug delivery systems with reasonable method, thus to prepare the multiple-unit drug delivery system of compound Niu Huang Xing Xiao pill, which achieves a better curative effect with reduced dosage.

The following is detailed description of the present invention in combination with embodiments.

## EXAMPLE 1 Preparation of the traditional Chinese medicine antitumor composition

A traditional Chinese medicine antitumor composition, comprising the following raw medicine materials by weight percentage: 32% of solid lipid nanoparticles containing volatile oil of frankincense and myrrh (FMO-SLN), 63% of nano realgar carrying microcapsule,1 % of bezoar micro-powder and 4% of musk micro-powder.

Preparation of the traditional Chinese medicine antitumor composition comprises the following steps:

(1) Extracting the frankincense and myrrh volatile oil component (FMO) with steam distillation method at first, and preparing the solid lipid nanoparticles containing volatile oil of frankincense and myrrh (FMO-SLN) with high pressure homogenization method.

Taking same amount of frankincense and myrrh respectively, crushing and mixing them together, extracting the volatile oil with water (8 times the amount of frankincense and myrrh) for 7 hours, and dehydrating the extracted FMO with anhydrous Na$_2$SO$_4$; preparing FMO-SLN with high pressure homogenization method: heating prescription amount of FMO (2%) together with glyceryl behenate (Compritol 888 ATO, 3%) in a water bath till temperature thereof is higher than its melting point temperature of 5°C, and obtaining oil phase after stirring and melting; placing and mixing prescription amount of soybean phospholipid and Tween-80 (Tween-80 : soybean phospholipid = 1:1,together 5%) in a beaker, adding proper amount of distilled water into the beaker, stirring for the mixture to dissolve, and heating till the temperature is the same as that of the oil phase to obtain the water phase; while being stirred by a constant-temperature magnetic stirrer, using a constant flow pump to add the water phase slowly into the oil phase, continuously stirring for a period of time to obtain the primary dispersed system, and then carrying out high pressure homogenization to obtain FMO-SLN. The average particle size is 113.3±3.6 nm, and the particle size distribution is narrow, from 43.8 nm to 219.5 nm; PDI is 0.25, and zeta potential is -16.8±0.4mV.

(2) Preparing nano realgar by using a high-energy ball mill with wet grinding method, and preparing nano realgar carrying microcapsule with gelatin and liquid paraffin and so on.

Crushing block-shaped realgar and sieving to obtain coarse particles, adding the coarse particles into a ball mill according to a ratio of grinding media to material of 40:1, and adding 10ml of saturated SDS solution there into as medium to carry out ball milling, rotating speed of the ball mill being 400r/min, rotating forwards for 30min, stopping for 3min, and rotating reversely for 30min, taking the realgar out after milling for 12h, carrying out water grind processing to obtain the nano realgar. Taking a proper amount of gelatin by precisely weighing, adding 20ml of distilled water to swelling the gelatin, carrying out heat preservation for dissolving, adding nano realgar there into according to a ratio of nano realgar to gelatin of 2:4, sufficiently stirring at a rotating speed of 700 r/min, taking 1g of Span-60 and adding it into 50ml of liquid paraffin; while continuously stirring, adding the gelatin solution containing medicine slowly into the liquid paraffin, after stirring in a water bath at a temperature of 40°C for a period of time, cooling it quickly so the temperature is less than 4°C; continuously stirring it in an ice-water bath for 1 h, adding 20ml of formaldehyde for curing, and then carrying out dehydration with 40ml of isopropanol, after standing in a refrigerator for 24h, carrying out suction filtration, and washing with a small quantity of isopropanol and normal hexane, pumping and drying in a stove at a temperature of 40°C, thus to obtain the flavescent nano realgar carrying microcapsule. Average particle size of the nano realgar is 85.4±3.5nm, and zeta potential is -34.3±1.7mV; more than 70% of the nano realgar carrying microcapsules are distributed at 90-125μm (standard sieve specified in Chinese Pharmacopoeia being 120-170 meshes).

(3) Preparing bezoar micro-powder by using a ball mill (dry grinding method). In the preparation technique, ratio of grinding media to material is 20:4, ball milling time is 90min, rotating speed of the ball mill is 300r/min, and the particle size is $14.46\pm1.93\mu m$.

(4) Preparing the artificial musk micro-powder by liquid nitrogen refrigeration and super centrifugal grinding method. Taking proper amount of artificial musk and freezing in the liquid nitrogen, taking it out and using super centrifugal grinding device to crush, thus to obtain artificial musk micro-powder, and the particle size is $32.53\pm3.6\mu m$.

(5) Adding proper amount of lactose into the above four units, sieving the medicine materials and auxiliary materials with an 80-mesh sieve, mixing uniformly and filling into No.1 capsule, thus to obtain the compound multiple-unit capsules.

**EXAMPLE 2 Preparation of the traditional Chinese medicine antitumor composition**

A traditional Chinese medicine antitumor composition, comprising the following raw medicine materials by weight percentage: 28% of solid lipid nanoparticles containing volatile oil of frankincense and myrrh (FMO-SLN), 61% of nano realgar carrying microcapsule, 0.8% of bezoar micro-powder and 6% of musk micro-powder.
Preparation of the traditional Chinese medicine antitumor composition comprises the following steps:

(1) Extracting the frankincense and myrrh volatile oil component (FMO) with steam distillation method at first, and preparing the solid lipid nanoparticles containing volatile oil of frankincense and myrrh (FMO-SLN) with high pressure homogenization method.

Taking same amount of frankincense and myrrh respectively, crushing and mixing them together, extracting the volatile oil with water (8 times the amount of frankincense and myrrh) for 7 hours, and dehydrating the extracted FMO with anhydrous $Na_2SO_4$; preparing FMO-SLN with high pressure homogenization method: heating prescription amount of FMO (2%) together with glyceryl behenate (Compritol 888 ATO, 3%) in a water bath till temperature thereof is higher than its melting point temperature of 5°C, and obtaining oil phase after stirring and melting; placing and mixing prescription amount of soybean phospholipid and Tween-80 (Tween-80 : soybean phospholipid = 1:1,together 5%) in a beaker, adding proper amount of distilled water into the beaker, stirring for the mixture to dissolve, and heating till the temperature is the same as that of the oil phase to obtain the water phase; while being stirred by a constant-temperature magnetic stirrer, using a constant flow pump to add the water phase slowly into the oil phase, continuously stirring for a period of time to obtain the primary dispersed system, and then carrying out high pressure homogenization to obtain FMO-SLN. The average particle size is $113.3\pm3.6$ nm, and the particle size distribution is narrow, from 43.8 nm to 219.5 nm; PDI is 0.25, and zeta potential is $-16.8\pm0.4$mV.

(2) Preparing nano realgar by using a high-energy ball mill with wet grinding method, and preparing nano realgar carrying microcapsule with gelatin and liquid paraffin and so on.

Crushing block-shaped realgar and sieving to obtain coarse particles, adding the coarse particles into a ball mill according to a ratio of grinding media to material of 40:1, and adding 10ml of saturated SDS solution there into as medium to carry out ball milling, rotating speed of the ball mill being 400r/min, rotating forwards for 30min, stopping for 3min, and rotating reversely for 30min, taking the realgar out after milling for 12h, carrying out water grind processing to obtain the nano realgar. Taking a proper amount of gelatin by precisely weighing, adding 20ml of distilled water to swelling the gelatin, carrying out heat preservation for dissolving, adding nano realgar there into according to a ratio of nano realgar to gelatin of 2:4, sufficiently stirring at a rotating speed of 700 r/min, taking 1g of Span-60 and adding it into 50ml of liquid paraffin; while continuously stirring, adding the gelatin solution containing medicine slowly into the liquid paraffin, after stirring in a water bath at a temperature of 40°C for a period of time, cooling it quickly so the temperature is less than 4°C; continuously stirring it in an ice-water bath for 1 h, adding 20ml of formaldehyde for curing, and then carrying out dehydration with 40ml of isopropanol, after standing in a refrigerator for 24h, carrying out suction filtration, and washing with a small quantity of isopropanol and normal hexane, pumping and drying in a stove at a temperature of 40°C, thus to obtain the flavescent nano realgar carrying microcapsule. Average particle size of the nano realgar is $85.4\pm3.5$nm, and zeta potential is $-34.3\pm1.7$mV; more than 70% of the nano realgar carrying microcapsules are distributed at $90-125\mu m$ (standard sieve specified in Chinese Pharmacopoeia being 120-170 meshes).

(3) Preparing bezoar micro-powder by using a ball mill (dry grinding method). In the preparation technique, ratio of grinding media to material is 20:4, ball milling time is 90min, rotating speed of the ball mill is 300r/min, and the particle size is $14.46\pm1.93\mu m$.

(4) Preparing the artificial musk micro-powder by liquid nitrogen refrigeration and super centrifugal grinding method. Taking proper amount of artificial musk and freezing in the liquid nitrogen, taking it out and using super centrifugal grinding device to crush, thus to obtain artificial musk micro-powder, and the particle size is $32.53\pm3.6\mu m$.

(5) Adding proper amount of auxiliary materials of tablet into the above four units, sieving the medicine materials and auxiliary materials with an 80-mesh sieve, mixing uniformly and compressing prepared tablets.

**EXAMPLE 3 Preparation of the traditional Chinese medicine antitumor composition**

A traditional Chinese medicine antitumor composition, comprising the following raw medicine materials by weight percentage: 29% of solid lipid nanoparticles containing volatile oil of frankincense and myrrh (FMO-SLN), 65% of nano realgar carrying microcapsule, 1.3% of bezoar micro-powder and 4.5% of musk micro-powder.

Preparation of the traditional Chinese medicine antitumor composition comprises the following steps:

(1) Extracting the frankincense and myrrh volatile oil component (FMO) with steam distillation method at first, and preparing the solid lipid nanoparticles containing volatile oil of frankincense and myrrh (FMO-SLN) with high pressure homogenization method.

Taking same amount of frankincense and myrrh respectively, crushing and mixing them together, extracting the volatile oil with water (8 times the amount of frankincense and myrrh) for 7 hours, and dehydrating the extracted FMO with anhydrous $Na_2SO_4$; preparing FMO-SLN with high pressure homogenization method: heating prescription amount of FMO (2%) together with glyceryl behenate (Compritol 888 ATO, 3%) in a water bath till temperature thereof is higher than its melting point temperature of 5°C, and obtaining oil phase after stirring and melting; placing and mixing prescription amount of soybean phospholipid and Tween-80 (Tween-80 : soybean phospholipid = 1:1,together 5%) in a beaker, adding proper amount of distilled water into the beaker, stirring for the mixture to dissolve, and heating till the temperature is the same as that of the oil phase to obtain the water phase; while being stirred by a constant-temperature magnetic stirrer, using a constant flow pump to add the water phase slowly into the oil phase, continuously stirring for a period of time to obtain the primary dispersed system, and then carrying out high pressure homogenization to obtain FMO-SLN. The average particle size is $113.3\pm3.6$ nm, and the particle size distribution is narrow, from 43.8 nm to 219.5 nm; PDI is 0.25, and zeta potential is $-16.8\pm0.4mV$.

(2) Preparing nano realgar by using a high-energy ball mill with wet grinding method, and preparing nano realgar carrying microcapsule with gelatin and liquid paraffin and so on.

Crushing block-shaped realgar and sieving to obtain coarse particles, adding the coarse particles into a ball mill according to a ratio of grinding media to material of 40:1, and adding 10ml of saturated SDS solution there into as medium to carry out ball milling, rotating speed of the ball mill being 400r/min, rotating forwards for 30min, stopping for 3min, and rotating reversely for 30min, taking the realgar out after milling for 12h, carrying out water grind processing to obtain the nano realgar. Taking a proper amount of gelatin by precisely weighing, adding 20ml of distilled water to swelling the gelatin, carrying out heat preservation for dissolving, adding nano realgar thereinto according to a ratio of nano realgar to gelatin of 2:4, sufficiently stirring at a rotating speed of 700 r/min, taking 1g of Span-60 and adding it into 50ml of liquid paraffin; while continuously stirring, adding the gelatin solution containing medicine slowly into the liquid paraffin, after stirring in a water bath at a temperature of 40°C for a period of time, cooling it quickly so the temperature is less than 4°C; continuously stirring it in an ice-water bath for 1 h, adding 20ml of formaldehyde for curing, and then carrying out dehydration with 40ml of isopropanol, after standing in a refrigerator for 24h, carrying out suction filtration, and washing with a small quantity of isopropanol and normal hexane, pumping and drying in a stove at a temperature of 40°C, thus to obtain the flavescent nano realgar carrying microcapsule. Average particle size of the nano realgar is $85.4\pm3.5nm$, and zeta potential is $-34.3\pm1.7mV$; more than 70% of the nano realgar carrying microcapsules are distributed at 90-125$\mu m$ (standard sieve specified in Chinese Pharmacopoeia being 120-170 meshes).

(3) Preparing bezoar micro-powder by using a ball mill (dry grinding method). In the preparation technique, ratio of grinding media to material is 20:4, ball milling time is 90min, rotating speed of the ball mill is 300r/min, and the particle size is $14.46\pm1.93\mu m$.

(4) Preparing the artificial musk micro-powder by liquid nitrogen refrigeration and super centrifugal grinding method. Taking proper amount of artificial musk and freezing in the liquid nitrogen, taking it out and using super centrifugal grinding device to crush, thus to obtain artificial musk micro-powder, and the particle size is $32.53\pm3.6\mu m$.

(5) Adding proper amount of auxiliary materials into the above four units, sieving the medicine materials and auxiliary materials with an 80-mesh sieve, mixing uniformly and producing granule.

Evaluating and characterizing the solid lipid nanoparticles containing volatile oil of frankincense and myrrh (FMO-SLN), nano realgar, nano realgar carrying microcapsule, bezoar micro-powder and musk micro-powder prepared in the examples 1~3 as follows:

I. Transmission electron microscope observation of FMO-SLN

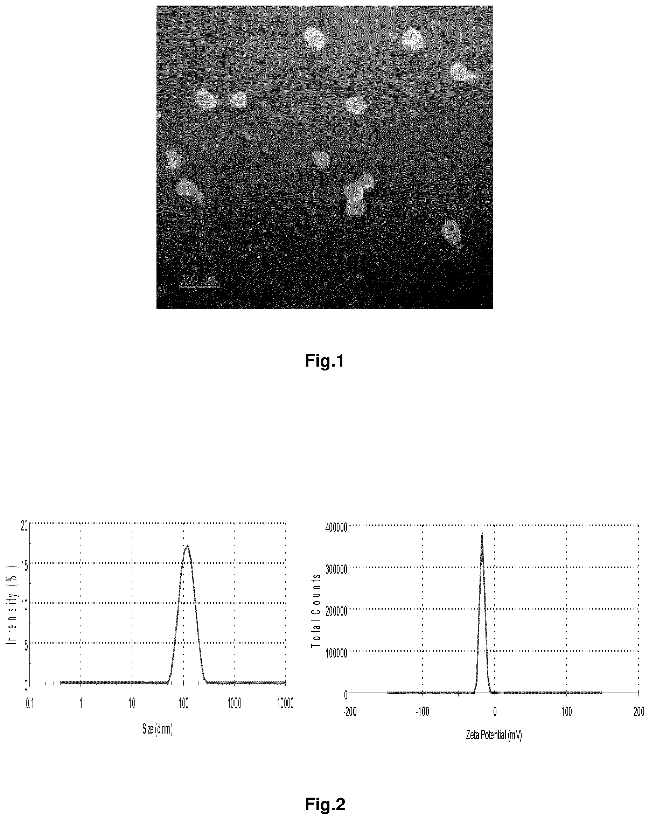

After being diluted with proper amount of distilled water, one drop of 2% phosphotungstic acid is added into FMO-SLN, and taking proper amount of the solution, and dropping it to a copper screen, naturally drying for about 10min, placing under the transmission electron microscope to observe, with result as shown in Fig.1, FMO-SLN being nearly spherical, and the size being uniform.

II. Measurement of particle size and potential

The prepared FMO-SLN is diluted with proper amount of deionized water, using Malvern particle size analyzer to measure the particle size and zeta potential, with result as shown in Fig.2, the average FMO-SLN particle size is $113.3\pm3.6$nm, the particle size distribution is narrow, from 43.8nm to 219.5nm, with PDI being 0.25. The zeta potential of FMO-SLN is $-16.8\pm0.4$mV, which shows that the surface of the FMO-SLN particle is negatively charged.

The average particle size of the nano realgar is $85.4\pm3.5$nm, and the zeta potential is $-34.3\pm1.7$mV; more than 70% of the nano realgar carrying microcapsules are distributed at 90-125$\mu$m (standard sieve specified in Chinese Pharmacopoeia being 120-170 meshes).

Particle size of the bezoar micro-powder is $14.46\pm1.93\mu$m.

Particle size of the musk micro-powder is $32.53\pm3.6\mu$m.

III. Measurement of encapsulation efficiency and drug loading capacity of the volatile oil of frankincense and myrrh (FMO) in FMO-SLN

According to measurement, in FMO-SLN, the encapsulation efficiency of the volatile oil of frankincense and myrrh (FMO) is 50% or higher, the average encapsulation efficiency being $(80.60\pm1.11)$%; the drug loading capacity of the volatile oil of frankincense and myrrh is 40%-60%, the average drug loading capacity being $(53.73\pm0.74)$%.

IV. Measurement of encapsulation efficiency and drug loading capacity of the nano realgar in the nano realgar carrying microcapsule

According to measurement, the encapsulation efficiency of the nano realgar in the nano realgar carrying microcapsule is 60% or higher, the average encapsulation efficiency being $(66.20\pm1.85)$%; the drug loading capacity of the nano realgar is 20%-40%, the average drug loading capacity being $(26.30\pm1.14)$%.

V. Transmission electron microscope observation of nano realgar

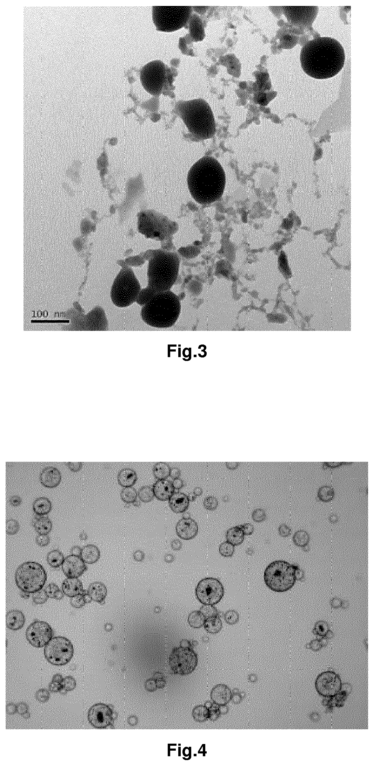

Using the transmission electron microscope to observe appearance of the nano realgar, after diluting the nano realgar with proper amount of distilled water, taking and dropping proper amount of solution to a copper screen, naturally drying for about 10min, placing under the transmission electron microscope to observe, with result as shown in Fig.3, the nano realgar being nearly spherical, and the size being uniform.

VI. Appearance observation of the nano realgar carrying microcapsule with electron microscope

Taking a small quantity of the nano realgar carrying microcapsule powder and placing the powder on glass slide, and dropping several drops of liquid paraffin, covering the cover glass and observing the appearance of microcapsule with electron microscope, as shown in Fig.4, the appearance of microcapsule is round and regular.

**EXAMPLE 4 $As_2S_2$ pharmacokinetics comparison after administration of the compound multiple-unit capsule with the Niu Huang Xing Xiao pill on the market**

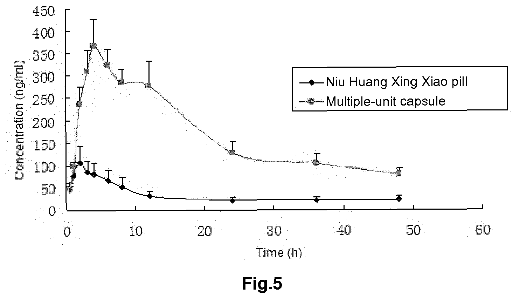

Beagle dogs are divided into two groups (n=4), namely Niu Huang Xing Xiao pill group (preparation on the market) and the compound multiple-unit capsule group prepared in Example 1, using gavage as administration method, Niu Huang Xing Xiao pill being filled into a capsule before gavage administration. The administration concentration according to original prescription is 90 mg/kg (concentration of realgar is about 15 mg/kg). After ministration, at the time point of 0.5h, 1.0h, 2.0h, 3.0h, 4.0h, 6.0h, 8.0h, 12.0h, 36.0h and 48h, sampling about 1.0ml of blood from vein of forelimb, and placing the sample in a heparin centrifuge tube, taking $200\mu l$ of plasma after centrifugation, carrying out microwave digestion and measuring content of $As_2S_2$ using hydride atomic absorption spectrometry, and calculating drug concentration in the plasma at each time point. Pharmacokinetic software 3P97 is used to process the pharmacokinetic parameters. Blood concentration - time curve is as shown in Fig.5, and the pharmacokinetic parameters are as shown in Table 1. After administration, compared with the Niu Huang Xing Xiao pill on the market, Cmax and AUC values of the multiple-unit capsule of the present invention increase remarkably (P<0.05), which shows that the bioavailability of the multiple-unit capsule is improved obviously. The parameters A, Ke, and CL/F indicate that absorption phase of the multiple-unit capsule group increases while elimination phase decreases. Ratios of Ka, $T_{1/2}$ and Tmax of the two groups are respectively 0.34:1, 1.96:1 and 1.99:1; $T_{1/2}$ and Tmax are obviously prolonged, which indicates that $As_2S_2$ in the multiple-unit capsule has a certain slow-release effect in vivo.

Table 1 Pharmacokinetic parameters

| Parameter | Unit | Niu Huang Xing Xiao pill | Multiple-unit capsule |
|---|---|---|---|
| A | ug/L | $115.87\pm35.37$ | $446.20\pm58.83a$ |
| Ka | 1/h | $1.99\pm0.13$ | $0.67\pm0.15a$ |
| Ke | 1/h | $0.090\pm0.023$ | $0.044\pm0.008a$ |
| $T_{1/2}$ | h | $8.24\pm2.38$ | $16.19\pm2.84a$ |
| CL/F | L/h | $49.59\pm24.35$ | $6.01\pm1.07a$ |
| Tmax | h | $2.17\pm0.41$ | $4.33\pm0.82a$ |
| Cmax | ug/L | $107.7\pm34.79$ | $368.55\pm56.94a$ |
| AUC(0-t) | $\mu$gh/L | $1566.26\pm468.99$ | $8164.85\pm1204.99a$ |

Note: aCompared with the Niu Huang Xing Xiao pill group, P<0.05.

The result shows that, compared with the Niu Huang Xing Xiao pill group, bioavailability of the compound multiple-unit capsule of the present invention is improved obviously.

**EXAMPLE 5 In-vivo tumor inhibitory effect comparison of the compound multiple-unit capsule with the Niu Huang Xing Xiao pill on the market**

The tumor-bearing Kunming mice are used to evaluate the in-vivo tumor inhibitory effect of the compound multiple-unit capsule of the present invention. Inoculating H22 hepatoma carcinoma cells (provided by Shanghai Institute of Materia Medica of Chinese Academy of Sciences) to enterocoelia of mice, taking out the H22 cells cultivated in the enterocoelia of mice after 6-8 days and preparing into homogeneous suspension with PBS; subcutaneously inoculating 0.2mL of H22 cells ($2 \times 10^6$/mL) to right axilla of each mouse. After inoculation, the mice are divided into 5 groups, 10 mice for each group: (1) Normal saline group (blank control), (2) 5-FU group (positive control), (3) 0.5% CMC-Na group, (4) Niu Huang Xing Xiao pill group and (5) Compound multiple-unit capsule group. The 5-FU group is subjected to administration through intraperitoneal injection, once every two days, with the dosage being 25 mg/kg. And the other four groups are subjected to intragastric administration. Preparation method of the Niu Huang Xing Xiao pill group and the compound multiple-unit capsule group is as follows: taking prescription amount of each component, adding the components into 0.5% CMC-Na water solution to prepare suspension. The concentration of drug for administration according to the original prescription for the groups (4) and (5) is 450 mg/kg (with the concentration of realgar being about 75 mg/kg). The tumor inhibiting rate is calculated with the following formula:

$$\text{Tumor inhibiting rate} = (C-T)/C \times 100\%;$$

C and T being average weight of the tumors on mice of the blank control and the treatment group.

The result is as shown in the following Table 2, compared with the Niu Huang Xing Xiao pill group, the tumor inhibiting rate of the compound multiple-unit capsule of the present invention is higher.

Table 2 In-vivo tumor inhibitory effect experimental result (n=10)

| Group | Weight of mice (g) | | Weight of tumor (g) | Tumor inhibiting rate (%) |
| --- | --- | --- | --- | --- |
| | Before | After | | |
| Normal saline | 19.80 ± 1.03 | 28.00±2.00 | 1.55 ±0.20 | - |
| 0.5% CMC-Na | 19.90 ± 1.20 | 27.60 ± 1.89 | 1.56 ± 0.24 | -0.50 |
| 5-FU (25 mg/kg) | 20.20 ± 0.92 | 24.20 ± 1.75 | 0.50 ± 0.13** | 67.95 |
| Niu Huang Xing Xiao pill | 19.70 ± 1.34 | 24.70±2.11 | 0.78±0.14** | 49.55 |
| Multiple-unit capsule | 19.90 ± 1.20 | 23.90 ± 2.42 | 0.66±0.17**# | 57.59 |

Note: **$P < 0.01$ Compared with the Normal saline group; #$P < 0.05$ compared with the Niu Huang Xing Xiao pill group.

The above embodiments are only ways of implementing the present invention, the description is specific and detailed, but it cannot be regarded as limitation to the scope of the present invention. It should be noted that the persons skilled in the art, without departing from the conception of the present invention, can further figure out modification and improvement, which shall all belong to the protective scope of the present invention. Therefore, the protective scope of the present invention shall be determined by the terms of the claims.

**Claims**

1.  A traditional Chinese medicine antitumor composition, comprising the following components by weight percentage: 28%-32% of solid lipid nanoparticles containing volatile oil of frankincense and myrrh, 61%-65% of nano realgar carrying microcapsule, 0.8%-1.3% of bezoar micro-powder and 4%-6% of musk micro-powder.

2.  The traditional Chinese medicine antitumor composition according to claim 1, wherein the particle size of the solid lipid nanoparticles containing volatile oil of frankincense and myrrh is 40-300nm.

3.  The traditional Chinese medicine antitumor composition according to claim 1, wherein the particle size of the nano realgar is 80-200nm.

4.  The traditional Chinese medicine antitumor composition according to claim 1, wherein the particle size of the bezoar micro-powder is 1-20$\mu$m.

5.  The traditional Chinese medicine antitumor composition according to claim 1, wherein the particle size of the musk micro-powder is 5-50$\mu$m.

6.  A method for preparation of the traditional Chinese medicine antitumor composition according to claim 1,

    - wherein the solid lipid nanoparticles containing volatile oil of frankincense and myrrh is prepared with the following method: extracting the volatile oil in the frankincense and myrrh with steam distillation method at first, and then preparing the solid lipid nanoparticles containing volatile oil of frankincense and myrrh with high pressure homogenization method;
    - wherein the nano realgar carrying microcapsule is prepared with the following method: preparing nano realgar by using a high-energy ball mill with wet grinding method at first, mixing the nano realgar with gelatin solution uniformly, and then adding the gelatin solution containing the nano realgar into liquid paraffin, mixing in a water bath, cooling and continuously mixing in an ice-water bath, adding formaldehyde for curing, and then carrying out dehydration with isopropanol, after standing for a certain time, carrying out suction filtration, and washing with isopropanol and normal hexane, pumping and drying, thus to obtain the nano realgar carrying microcapsule;
    - wherein the bezoar micro-powder is prepared by using a ball mill with dry grinding method; and
    - wherein the musk micro-powder is prepared with combination of liquid nitrogen refrigeration and super centrifugal grinding method.

7.  The traditional Chinese medicine antitumor composition prepared according to claim 6, wherein the encapsulation efficiency of the mixed volatile oil of frankincense and myrrh in the solid lipid nanoparticles containing volatile oil of frankincense and myrrh is 50% or higher.

8. The traditional Chinese medicine antitumor composition prepared according to claim 6, wherein the drug loading capacity of the mixed volatile oil of frankincense and myrrh in the solid lipid nanoparticles containing volatile oil of frankincense and myrrh is 40%-60%.

9. The traditional Chinese medicine antitumor composition prepared according to claim 7, wherein the encapsulation efficiency of the nano realgar in the nano realgar carrying microcapsule is 60% or higher.

10. The traditional Chinese medicine antitumor composition prepared according to claim 7, wherein the drug loading capacity of the nano realgar in the nano realgar carrying microcapsule is 20%-40%.

11. The traditional Chinese medicine antitumor composition according to claim 1, wherein the dosage form of the traditional Chinese medicine antitumor composition includes capsule, granule or tablet.

12. The traditional Chinese medicine antitumor composition according to claim 1 for use in the treatment of tumors.


**Patentansprüche**

1. Antitumor-Zusammensetzung der traditionellen chinesischen Medizin, umfassend die folgenden Bestandteile in Gewichtsprozent: 28 % bis 32 % feste Lipid-Nanoteilchen, die flüchtiges Öl von Weihrauch und Myrrhe enthalten, 61 % bis 65 % Mikrokapsel, die Nano-Realgar fasst, 0,8 % bis 1,3 % Bezoar-Mikropulver und 4 % bis 6 % Moschus-Mikropulver.

2. Antitumor-Zusammensetzung der traditionellen chinesischen Medizin nach Anspruch 1, wobei die Teilchengröße der festen Lipid-Nanoteilchen, die flüchtiges Öl von Weihrauch und Myrrhe enthalten, 40 bis 300 nm beträgt.

3. Antitumor-Zusammensetzung der traditionellen chinesischen Medizin nach Anspruch 1, wobei die Teilchengröße des Nano-Realgars 80 bis 200 nm beträgt.

4. Antitumor-Zusammensetzung der traditionellen chinesischen Medizin nach Anspruch 1, wobei die Teilchengröße des Bezoar-Mikropulvers 1 bis 20 $\mu$m beträgt.

5. Antitumor-Zusammensetzung der traditionellen chinesischen Medizin nach Anspruch 1, wobei die Teilchengröße des Moschus-Mikropulvers 5 bis 50 $\mu$m beträgt.

6. Verfahren zur Herstellung der Antitumor-Zusammensetzung der traditionellen chinesischen Medizin nach Anspruch 1,

   - wobei die festen Lipid-Nanoteilchen, die flüchtiges Öl von Weihrauch und Myrrhe enthalten, durch das folgende Verfahren hergestellt werden: zuerst Extrahieren des flüchtigen Öls in dem Weihrauch und der Myrrhe mithilfe eines Dampfdestillationsverfahrens und anschließend Herstellen der festen Lipid-Nanoteilchen, die flüchtiges Öl von Weihrauch und Myrrhe enthalten, mithilfe eines Hochdruck-Homogenisierungsverfahrens;
   - wobei die Mikrokapsel, die Nano-Realgar fasst, mithilfe des folgenden Verfahrens hergestellt wird: zuerst Herstellen von Nano-Realgar unter Benutzen einer Hochenergie-Kugelmühle mithilfe eines Nassschleifverfahrens, Mischen des Nano-Realgars mit Gelatinelösung gleichförmig und anschließend Eingeben der Gelatinelösung, die das Nano-Realgar enthält, in flüssiges Paraffin, Mischen in einem Wasserbad, Kühlen und kontinuierliches Mischen in einem Eiswasserbad, Zusetzen von Formaldehyd zum Härten und anschließend Durchführen von Wasserentziehung mit Isopropanol, nach dem Stehen während eines bestimmten Zeitraums, Durchführen von Saugfiltration und Waschen mit Isopropanol und Normal-Hexan, Pumpen und Trocknen, um so die Mikrokapsel, die Nano-Realgar fasst, zu gewinnen;
   - wobei das Bezoar-Mikropulver mithilfe eines Trockenschleifverfahrens unter Benutzen einer Kugelmühle hergestellt wird; und
   - wobei das Moschus-Mikropulver mithilfe einer Kombination von Kühlung mit flüssigem Stickstoff und Superfliehkraft-Schleifverfahren hergestellt wird.

7. Antitumor-Zusammensetzung der traditionellen chinesischen Medizin, hergestellt nach Anspruch 6, wobei die Kapselungseffizienz des gemischten flüchtigen Öls von Weihrauch und Myrrhe in den festen Lipid-Nanoteilchen, die flüchtiges Öl von Weihrauch und Myrrhe enthalten, 50 % oder höher ist.

**8.** Antitumor-Zusammensetzung der traditionellen chinesischen Medizin, hergestellt nach Anspruch 6, wobei die Arzneimittel-Beladungskapazität an dem gemischten flüchtigen Öl von Weihrauch und Myrrhe in den festen Lipid-Nanoteilchen, die flüchtiges Öl von Weihrauch und Myrrhe enthalten, 40 % bis 60 % beträgt.

**9.** Antitumor-Zusammensetzung der traditionellen chinesischen Medizin, hergestellt nach Anspruch 7, wobei die Kapselungseffizienz des Nano-Realgars in der Mikrokapsel, die Nano-Realgar fasst, 60 % oder höher ist.

**10.** Antitumor-Zusammensetzung der traditionellen chinesischen Medizin, hergestellt nach Anspruch 7, wobei die Arzneimittel-Beladungskapazität an dem Nano-Realgar in der Mikrokapsel, die Nano-Realgar fasst, 20 % bis 40 % beträgt.

**11.** Antitumor-Zusammensetzung der traditionellen chinesischen Medizin nach Anspruch 1, wobei die Darreichungsform der Antitumor-Zusammensetzung der traditionellen chinesischen Medizin u.a. Kapsel, Granalie oder Tablette ist.

**12.** Antitumor-Zusammensetzung der traditionellen chinesischen Medizin nach Anspruch 1 zur Benutzung in der Behandlung von Tumoren.

**Revendications**

**1.** Composition antitumorale de la médecine chinoise traditionnelle, comprenant les composants suivants en pourcentage en poids : 28 à 32 % de nanoparticules lipidiques solides contenant de l'huile volatile d'encens et de myrrhe, 61 à 65 % de microcapsules portant du nano réalgar, 0,8 à 1,3 % de micro-poudre de bézoard et 4 à 6 % de micro-poudre de musc.

**2.** Composition antitumorale de la médecine chinoise traditionnelle selon la revendication 1, dans laquelle la taille de particule des nanoparticules lipidiques solides contenant de l'huile volatile d'encens et de myrrhe est de 40 à 300 nm.

**3.** Composition antitumorale de la médecine chinoise traditionnelle selon la revendication 1, dans laquelle la taille de particule du nano réalgar est de 80 à 200 nm.

**4.** Composition antitumorale de la médecine chinoise traditionnelle selon la revendication 1, dans laquelle la taille de particule de la micro-poudre de bézoard est de 1 à 20 mm.

**5.** Composition antitumorale de la médecine chinoise traditionnelle selon la revendication 1, dans laquelle la taille de particule de la miro-poudre de musc est de 5 à 50 mm.

**6.** Procédé de préparation de la composition antitumorale de la médecine chinoise traditionnelle selon la revendication 1,

- dans laquelle les nanoparticules lipidiques solides contenant de l'huile volatile d'encens et de myrrhe sont préparées avec le procédé suivant : extraction de l'huile volatile dans l'encens et la myrrhe avec un procédé de distillation de vapeur en premier, puis préparation des nanoparticules lipidiques solides contenant de l'huile volatile d'encens et de myrrhe avec un procédé d'homogénéisation à haute pression ;
- dans laquelle les microcapsules portant le nano réalgar sont préparées avec le procédé suivant : préparation de nano réalgar en utilisant un broyeur à boule de haute énergie avec un procédé de broyage humide en premier, mélange du nano réalgar avec une solution de gélatine uniformément, puis ajout de la solution de gélatine contenant le nano réalgar dans de la paraffine liquide, mélange dans un bain d'eau, refroidissement et mélange en continu dans un bain d'eau glacée, ajout de formaldéhyde pour le durcissement, puis réalisation d'une déshydratation avec de l'isopropanol, après repos durant un certain temps, réalisation d'une filtration par succion, et lavage avec de l'isopropanol et de l'hexane normal, pompage et séchage, pour obtenir ainsi les microcapsules portant le nano réalgar ;
- dans laquelle la micro-poudre de bézoard est préparée en utilisant un broyeur à boule avec un procédé de broyage à sec ; et
- dans laquelle la micro-poudre de musc est préparée par combinaison de réfrigération à l'azote liquide et de procédé de broyage à super centrifugeuse.

**7.** Composition antitumorale de la médecine chinoise traditionnelle préparée selon la revendication 6, dans laquelle l'efficacité d'encapsulation de l'huile volatile mixte d'encens et de myrrhe dans les nanoparticules lipidiques solides contenant de l'huile volatile d'encens et de myrrhe est de 50 % ou plus.

8.  Composition antitumorale de la médecine chinoise traditionnelle préparée selon la revendication 6, dans laquelle la capacité de chargement de médicament de l'huile volatile mixte d'encens et de myrrhe dans les nanoparticules lipidiques solides contenant de l'huile volatile d'encens et de myrrhe est de 40 à 60 %.

9.  Composition antitumorale de la médecine chinoise traditionnelle préparée selon la revendication 7, dans laquelle l'efficacité d'encapsulation du nano réalgar dans les microcapsules portant le nano réalgar est de 60 % ou plus.

10. Composition antitumorale de la médecine chinoise traditionnelle préparée selon la revendication 7, dans laquelle la capacité de chargement de médicament du nano réalgar dans les microcapsules portant le nano réalgar est de 20 à 40 %.

11. Composition antitumorale de la médecine traditionnelle chinoise selon la revendication 1, dans laquelle la forme posologique de la composition antitumorale de la médecine traditionnelle chinoise comprend une capsule, un granulé ou un comprimé :

12. Composition antitumorale de la médecine traditionnelle chinoise selon la revendication 1 pour une utilisation dans le traitement de tumeurs.

**Fig.1**

**Fig.2**

**Fig.3**

**Fig.4**

**Fig.5**

**EP 2 845 599 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 1362141